# EUROPEAN PATENT SPECIFICATION

(11) **EP 1 268 545 B1**
(45) Date of publication and mention of the grant of the patent: **16.08.2006**
(21) Application number: 01909831.8
(22) Date of filing: 13.03.2001
(51) Int. Cl.: C07K 14/705, C12N 15/12, C12N 15/63, C12N 5/10

(54) **POTASSIUM CHANNEL PROTEIN KCNQ5, A TARGET FOR DISEASES OF CENTRAL NERVOUS SYSTEM AND CARDIOVASCULAR SYSTEM**
KALIUMKANALPROTEIN KCNQ5, EIN ZIEL FÜR ERKRANKUNGEN DES ZENTRALEN NERVENSYSTEMS UND HERZ-GEFÄSS-SYSTEMS
PROTEINE DU CANAL CALCIQUE KCNQ5, CIBLE DE MALADIES DU SYSTEME NERVEUX CENTRAL ET DU SYSTEME CARDIOVASCULAIRE

(30) Priority: 21.03.2000 DE 10013732
(43) Date of publication of application: 02.01.2003
(73) Proprietor: Sanofi-Aventis Deutschland GmbH, 65929 Frankfurt am Main (DE)
(72) Inventor: LERCHE, Christian, 40221 Düsseldorf (DE); SCHERER, Constanze, 72076 Tübingen (DE); SEEBOHM, Guiscard, 37647 Polle (DE); BUSCH, Andreas, 65779 Kelkheim (DE); STEINMEYER, Klaus, 60323 Frankfurt (DE)
(86) International application number: PCT/EP2001/002791
(87) International publication number: WO 2001/070811

(56) References cited:
- WO-A-00/44786
- WO-A-00/61606
- WO-A-00/77035
- WO-A-99/07832
- WO-A-99/21875
- WO-A-99/31232
- C. LERCHE ET AL., : "Molecular cloning and functional expression of KCNQ5, a potassium channel subunit that may contribute to neuronal M-current diversity " JOURNAL BIOLOGICAL CHEMISTRY, vol. 275, no. 29, 21 July 2000 (2000-07-21), pages 22395-22400, XP002169157
- B.C. SCHROEDER ET AL., : "KCNQ5, a novel potassium channel broadly expressed in brain, mediates M-type currents" JOURNAL BIOLOGICAL CHEMISTRY, vol. 275, no. 31, 4 August 2000 (2000-08-04), pages 24089-24095, XP002169158

### SEQUENCE LISTING

<110> Aventis Pharma Deutschland GmbH
<120> Potassium channel protein KCNQ5, a new target for diseases of central nervous system and cardiovascular system
<130> AVE D-2000/A019
<140>
   <141>
<150> 10013732.6
   <151> 2000-03-21
<160> 2
<170> PatentIn Ver. 2.1
<210> 1
   <211> 3074
   <212> DNA
   <213> Homo sapiens
<400> 1
   gggcgccccg tcggccgccg gcttcctcct tgaaacccgc cggcgcacat gaggccgctg 60
   cccccgccgc aggcgctggc ggccccctcg cggtgcccgt ggtgatgcca tgccccgcca 120
   ccacgcggga ggagaggagg gcggcgccgc cgggctctgg gtgaagagcg gcgcagcggc 180
   ggcggcggcg ggcggggggc gcttgggcag cggcatgaag gatgtggagt cgggccgggg 240
   cagggtgctg ctgaactcgg cagccgccag gggcgacggc ctgctactgc tgggcacccg 300
   cgcggccacg cttggtggcg gcggcggtgg cctgagggag agccgccggg gcaagcaggg 360
   ggcccggatg agcctgctgg gaagccgcct ctcttacacg agtagccaga gctgccggcg 420
   caacgtcaag taccggcggg tgcagaacta cctgtacaac gtgctggaga gaccccgcgg 480
   ctgggcgttc atctaccacg ctttcgtttt cctccttgtc tttggttgct tgattttgtc 540
   agtgttttct accatccctg agcacacaaa attggcctca agttgcctct tgatcctgga 600
   gttcgtgatg attgtcgtct ttggtttgga gttcatcatt cgaatctggt ctgcgggttg 660
   ctgttgtcga tatagaggat ggcaaggaag actgaggttt gctcgaaagc ccttctgtgt 720
   tatagatacc attgttctta tcgcttcaat agcagttgtt tctgcaaaaa ctcagggtaa 780
   tatttttgcc acgtctgcac tcagaagtct ccgtttccta cagatcctcc gcatggtgcg 840
   catggaccga aggggaggca cttggaaatt actgggttca gtggtttatg ctcacagcaa 900
   ggaattaatc acagcttggt acataggatt tttggttctt attttttcgt ctttccttgt 960
   ctatctggtg gaaaaggatg ccaataaaga gctttctaca tatgcagatg ctctctggtg 1020
   gggcacaatt acattgacaa ctattggcta tggagacaaa actcccctaa cttggctggg 1080
   aagattgctt tctgcaggct ttgcactcct tggcatttct ttctttgcac ttcctgccgg 1140
   cattcttggc tcaggttttg cattaaaagt acaagaacaa caccgccaga aacactttga 1200
   gaaaagaagg aacccagctg ccaacctcat tcagtgtgtt tggcgtagtt acgcagctga 1260
   tgagaaatct gtttccattg caacctggaa gccacacttg aaggccttgc acacctgcag 1320
   ccctaccaag aaagaacaag gggaagcatc aagcagtcag aagctaagtt ttaaggagcg 1380
   agtgcgcatg gctagcccca ggggccagag tattaagagc cgacaagcct cagtaggtga 1440
   caggaggtcc ccaagcaccg acatcacagc cgagggcagt cccaccaaag tgcagaagag 1500
   ctggagcttc aacgaccgaa cccgcttccg gccctcgctg cgcctcaaaa gttctcagcc 1560
   aaaaccagtg atagatgctg acacagccct tggcactgat gatgtatatg atgaaaaagg 1620
   atgccagtgt gatgtatcag tggaagacct caccccacca cttaaaactg tcattcgagc 1680
   tatcagaatt atgaaatttc atgttgcaaa acggaagttt aaggaaacat tacgtccata 1740
   tgatgtaaaa gatgtcattg aacaatattc tgctggtcat ctggacatgt tgtgtagaat 1800
   taaaagcctt caaacacgtg ttgatcaaat tcttggaaaa gggcaaatca catcagataa 1860
   gaagagccga gagaaaataa cagcagaaca tgagaccaca gacgatctca gtatgctcgg 1920
   tcgggtggtc aaggttgaaa aacaggtaca gtccatagaa tccaagctgg actgcctact 1980
   agacatctat caacaggtcc ttcggaaagg ctctgcctca gccctcgctt tggcttcatt 2040
   ccagatccca ccttttgaat gtgaacagac atctgactat caaagccctg tggatagcaa 2100
   agatctttcg ggttccgcac aaaacagtgg ctgcttatcc agatcaacta gtgccaacat 2160
   ctcgagaggc ctgcagttca ttctgacgcc aaatgagttc agtgcccaga ctttctacgc 2220
   gcttagccct actatgcaca gtcaagcaac acaggtgcca attagtcaaa gcgatggctc 2280
   agcagtggca gccaccaaca ccattgcaaa ccaaataaat acggcaccca agccagcagc 2340
   cccaacaact ttacagatcc cacctcctct cccagccatc aagcatctgc ccaggccaga 2400
   aactctgcac cctaaccctg caggcttaca ggaaagcatt tctgacgtca ccacctgcct 2460
   tgttgcctcc aaggaaaatg ttcaggttgc acagtcaaat ctcaccaagg accgttctat 2520
   gaggaaaagc tttgacatgg gaggagaaac tctgttgtct gtctgtccca tggtgccgaa 2580
   ggacttgggc aaatctttgt ctgtgcaaaa cctgatcagg tcgaccgagg aactgaatat 2640
   acaactttca gggagtgagt caagtggctc cagaggcagc caagattttt accccaaatg 2700
   gagggaatcc aaattgttta taactgatga agaggtgggt cccgaagaga cagagacaga 2760
   cacttttgat gccgcaccgc agcctgccag ggaagctgcc tttgcatcag actctctaag 2820
   gactggaagg tcacgatcat ctcagagcat ttgtaaggca ggagaaagta cagatgccct 2880
   cagcttgcct catgtcaaac tgaaataagt tcttcatttt ctttccaggc atagcagttc 2940
   tttagccata catatcattg catgaactat ttcgaaagcc cttctaaaaa gttgaaattg 3000
   caagaatcgg gaagaacatg aaaggcagtt tataagcccg ttacctttta attgcatgaa 3060
   aatgcatgtt tagg                                                    3074
<210> 2
   <211> 932
   <212> PRT
   <213> Homo sapiens
<400> 2

## Description

The invention is related to a novel potassium channel protein KCNQ5 which is considered as target for diseases of central nervous system and cardiovascular system. The protein is used as screening tool for identification of compounds which improve symptoms of diseases of central nervous system and cardiovascular system.

Voltage-dependent potassium channels are key regulators of the resting membrane potential and modulate the excitability of electrically active cells like neurons and cardiac myocytes. Several classes of voltage-dependent K⁺ channels have been cloned and probably all form oligomeric proteins through the assembly of four α-protein subunits. The tetrameric pore complex further can interact with auxiliary subunits that enhance and/or modify currents mediated by the pore-forming α-subunits.

The KCNQ family of voltage-dependent K⁺ channels originally was established by positional cloning of the KCNQ1 gene (KvLQT1) which encodes a K⁺ channel protein with six transmembrane domains and a characteristic pore region. So far the KCNQ family consists of four members all of which are associated with human diseases. KCNQ1 functionally interacts with KCNE1, a small β-subunit protein with a single transmembrane domain, to generate the slowly-activating delayed-rectifier I_{Ks} current of cardiomyocytes. Inactivating mutations in both subunits result in the prolongation of the cardiac action potential and an increased risk of ventricular arrhythmias in patients with long QT-syndrome (LQTS). KCNQ1 and KCNE1 also are found in the inner ear and some loss of function mutations of KCNE1 and KCNQ1 are associated with hearing loss. In intestine KCNQ1 probably associates with the structurally related KCNE3 protein to generate different K⁺ channels. The KCNQ1/KCNE3 channel complex possibly represents the basolateral cAMP¹-regulated K⁺ conductance in colonic crypts probably important for apical cAMP-stimulated chloride secretion and that is involved in secretory diarrhoea and cystic fibrosis.

KCNQ2 and KCNQ3 are expressed in the brain and colocalize in various brain areas (Biervert, C. et al.; Science 279, 403-406, 1998). Whereas KCNQ2 generates K⁺ currents very similar to KCNQ1 (Schröder, B. C. et al.; Nature 396, 687-690, 1998), KCNQ3 alone produces only small currents (Wang, W.-P. et al., J. Biol. Chem. 273, 19419-19423, 1998). Coexpression of both KCNQ2 and KCNQ3 resulted in currents that were at least 10-fold larger than that of KCNQ2 alone (Wang, H.-S. et al.; Science 282, 1890-1893, 1998), suggesting that KCNQ3 facilitates expression of KCNQ2 subunits, by formation of a heteromeric complex of KCNQ2 and KCNQ3 subunits. The genes encoding KCNQ2 and KCNQ3 have been cloned due to their linkage to a form of epilepsy in human infants and loss of function mutations have been identified in both genes in patients with benign familial neonatal convulsions (BNFC) (Sigh, N. A. et al.; N. Genet. 18, 25-29, 1998; Charlier, C. et al.; N. Genet. 18, 53-55, 1998)). Since epilepsy is due to an electrical hyperexcitability in the brain, KCNQs may have an important stabilizing role in the nervous system. Biophysical and pharmacological properties of KCNQ2/KCNQ3 currents are very similar to that of the native neuronal M-type K⁺ current that is also characterized by muscarinic modulation and that is thought to be a prominent regulator of neuronal excitability as well. Similar to the native M-current, KCNQ2/KCNQ3 channel activity is strongly reduced by muscarinic acetylcholine agonists and therefore it is now assumed that KCNQ2 and KCNQ3 subunits contribute to the native M-channel.
KCNQ4, another member of this gene family, is expressed in sensory outer hair cells of the cochlea and is mutated in dominant deafness. Interestingly, coexpression of KCNQ3 with KCNQ4 also increased current amplitudes, although to a far less extent than observed with KCNQ2/KCNQ3 coexpression. This raises the possibility that different KCNQ channels can combine to produce variants of M-currents in different parts of the nervous system.

Problem of the invention was to identify another gene member of the KCNQ family including the protein for use as a target for treatment of various diseases.

KCNQ5 produces K⁺ channels that are activated by depolarization and that are very similar to the currents generated by other KCNQ channels. KCNQ5 is expressed in skeletal muscle and in the brain, where its expression pattern overlaps with that of KCNQ2 and KCNQ3 that underlie the native M-current. Similar to KCNQ2, KCNQ5. forms functional heteromers with KCNQ3, suggesting that neuronal M-channels probably can also include KCNQ5 subunits.

The subject matter of this invention is related to a DNA sequence coding for a polypeptide exhibiting the activity of a potassium voltage gated channel, wherein said polypeptide shall have the aminoacid sequence of SEQ ID No 2.

This invention is further related to a DNA sequence having the polynucleotide sequence of Seq ID No 1.

The invention refers further to a recombinant DNA vector, said vector comprising a polynucleotide element which renders the vector suitable for its multiplication in procaryotic or eucaryotic cells and a DNA sequence as aforementioned coding for the amino acid sequence of SEQ ID No.2 the polynucleotide sequence of SEQ ID No.1. This DNA element which renders the vector suitable for mulitiplication can be a origin of replication which works in procaryotiy or eucaryotic cells. An example for an origin of replication which works in procaryotic cells is the colE1 ori. A recombinant vector needs further a selection marker for control of growth of these organisms which harbor the vector. As selection markers suitable are genes which protect organism from antibiotics (antibioticum resistance) e.g. ampicillin, streptomycin, chloramphenicol or provide growth under compound deprived environmental conditions (auxotrophic growth conditions) when expressed as proteins in cells.

In a preferred embodiment of the invention for multiplication of the said recombinant vector the procaryotic cells are bacteria. In special preferred versions of the inventions he bacteria are in particular bacteria of Escherichia coli or of Bacillus spec.. In a further preferred embodiment of the invention for the multiplication of the said recombinant vector the eucaroytic cells are cells of a cell line or yeast cells. In special preferred versions of the invention the cells of the cell line are cells of a COS, Hela-, or 3T3-cell-line and the yeast cells are cells of Saccharomyces cerevisiae.

The said recombinant DNA could provide for a promotor element which is operationally linked to a DNA sequence coding for the amino acid sequence or polynucleotide sequence of a kCNQ5 allowing transcription of the related RNA and/or expression of the related protein. This promotor element an be taken in preferred versions of the invention from procaryotic promotors or eucaryotic promotors. A procaryotic is characterized by its ability to induce transcription in procaryotic organisms as a eucaryotic promotor is characterized by its ability to induce transcription in eucaryotic organisms. Both procaryotic and eucaryotic promotor element can be preferred incucible promotors or further preferred constitutive promotors. An inducible promotor is switched on only when a signal event is present. The signal can be bom by the organisms metabolism. Then it often consists of metabolic products, hormones, degradation products of macromolecules or other metabolic derived substances. The signal can also be provided by the environment.

Then it may consist of radiation, temperature or chemical compounds of the environmerit. A constitutive promotor needs no Induction for activity.

The invention includes further a host cell this host cell comprising at least one recombinant DNA vector as mentioned before. The host cell is taken preferred from procaryotic or eucaryotic cells. When it is taken from procaryotic cells it preferably consists of a cell of a bacterium in particular of Escherichia coli or Bacillus spec.. When this host cell consists of a eucaryotic cell it is preferred a cell of a cell line in particular a cell of a COS-, a Hela-, or 3T3-cell-line or a cell of a yeast in particular a cell of Saccaromyces cerevisiae.

This host cell can be produced by transforming the said host cell by a recombinant DNA vector comprising a DNA sequence coding for a amino acid sequence of SEQ ID No 2 or the polynucleotide sequence of SEQ ID No.1. The transformation can take place by routine methods used in microbiology as for example transformation of competent cells, Ca²⁺-phosphate-precipitation or electroporation.

The invention refers also to a protein encoded by one of the DNA sequences as aforementioned. This protein has activity of a KCNQ5. Activity of KCNQ5 is characterized by ion transport of this transporter type. Further included is production of a protein wherein first a host cell harboring a recombinant vector including a DNA sequence encoding for an amino acid sequence or a polynucleotide sequence for KCNQ5 is propagated in a suitable growth medium chosen from either media for bacteria or eucaryotic cells in dependance of the related cell type. This propagated cells are second harvested by common methods of biochemistry as centrifugation or filtration and processed to obtain crude cell extracts. These cell extracts third are purified subsequently by methods used for protein purification as size exchange chromatography, ion exhange chromatography, affinity chromatography and others to gain the protein of interest (KCNQS) separated from other compounds of the cell lysates.

The invention includes further a process for identification of a compound which compound is suitable for modification of activity of a protein having activity of a potassium voltage gated channel as mentioned before comprising
a) Providing of the above protein having activity of a potassium voltage gated channel ;
b) Providing of at least one chemical compound;
c) Incubating the chemical compound according to b) with a protein accoding to a);
d) Measuring activity of protein accoding to a).

In a preferred embodiment of the invention the above protein having activity of a potassium voltage gated channel as claimed from a) is provided within a host cell as aforementioned. The above protein having activity of may interact with other proteins for the purpose of this invention in a further preferred version of the invention. In particular it interacts with KCNQ3. Such a screening assay is applicable for identification of at least one chemical compound which modifies activity of KCNQ5 and which has Impact on diseases of central nervous system or cardiovascular system.

### Example:

### Example 1: Molecular Cloning and Expression of KCNQ5

KCNQ5 a novel member of the KCNQ family of potassium channels was isolated. The gene is expressed in different regions of the brain and in skeletal muscle. When expressed in Xenopus oocytes KCNQ5 alone generates voltage-dependent, slowly activating K⁺-selective currents that are insensitive to the K⁺ channel blocker TEA and display a marked inward rectification at positive membrane voltages. Upon coexpression with the related KCNQ3 channel amplitude of the K⁺ current is increased 4-5 fold. Compared to homomeric KCNQ5 currents, KCNQ3/KCNQ5 heteromeric currents also display slower activation and less inward rectification, showing that KCNQ5 combines with KCNQ3 to form functional channel proteins. The functional interaction between KCNQ5 and KCNQ3, a known protein subunit of the neuronal M-current, suggests that KCNQ5 possibly can contribute to native neuronal M-like currents.
The KCNQ5 gene was initially identified as a genomic survey sequence (BAC clone) in a homology search of GenBank (accession number AQ344243). Using this sequence a human brain cDNA library (available from Edge BioSystems) was screened. A composite full-length cDNA construct was assembled from two overlapping cDNA clones and subcloned into the Xenopus expression vector pSGEM (Villmann, C. et al.; J. Neurosci. 17, 7634-7643). The cDNA was fully sequenced on both strands using an automated DNA sequencer (ABI 310). For Xenopus oocyte expression capped cRNA was synthesized using the T7 mMessage mMachine kit (AMBION). For northern blot analysis a DIG-labeled riboprobe of 1.6 kb in length (containing mainly C-terminal sequences) was generated with the DIG RNA Labeling kit (available from ROCHE Diagnostics) according to the manufacturers instructions and hybridized to a series of human RNA blots (available from CLONTECH).

### Example 2: Chromosomal Localization of KCNQ5

Fluorescence In Situ Hybridization (available from FISH, Genome Systems) analysis was performed to determine the chromosomal localization of KCNQ5. Briefly, purified DNA from the BAC clone (AQ344243) was labeled with digoxigenin dUTP by nick translation and the labeled probe was hybridized to human metaphase chromosomes derived from PHA stimulated peripheral blood lymphocytes. KCNQ5-specific signals were detected on the long arm of chromosome 6 (6q14) using fluoresceinated antidigoxigenin antibodies followed by counterstaining with DAPI and confirmed by cohybridization with a genomic clone previously mapped to 6p21.

### Example 3: Electrophysiology

Xenopus laevis oocytes were obtained from tricaine anaesthetized animals. Ovaries were collagenase treated (1 mg/ 1 ml, Worthington, type II) in OR 2 solution (NaCl 82.5 mM, KCl 2 mM, MgCl₂ 1 mM, HEPES 5 mM, pH 7.4) for 120 min and subsequently stored in recording solution ND-96 (NaCl 96 mM, KCI 2 mM, CaCl₂ 1.8 mM, MgCl₂ 1 mM, HEPES 5 mM, pH 7.4) with additional NaPyruvate (275 mg/l), Theophylline (90 mg/l) and Gentamycin (50 mg/l) at 18°C. Oocytes were individually injected with 10 ng cRNA encoding hKCNQ5, rKCNQ3 (GenBank accession number AF087454), hKCNQ2, and hKCNQ1, respectively, or coinjected with 10 ng hKCNQ5 plus 5 ng hlsk (KCNE1), hMiRP1 (KCNE2), hMiRP2 (KCNE3), mMiRP3 (KCNE4), respectively. rKCNQ3 was cloned from a λZAP cDNA library (STRATAGENE) using an DIG-labeled (ROCHE) EST-clone (GenBank accession number AA019129) as a probe. hMiRP1 and hMiRP2 were cloned from human genomic DNA, mMiRP3 from murine brain cDNA using primers derived from the published sequences (13). Standard two-electrode voltage-clamp recordings were performed at room temperature with a Turbo Tec 10CD (NPI) amplifier, an ITC-16 interface combined with Pulse software (HEKA) and Origin for data aquisition on Pentium 11 PC. Macroscopic currents were recorded 2-4 days after injection. All fitting procedures were based on the simplex algorithm. Student's t test was used to test for statistical significance, which was assumed if P<0.05 and indicated by *.

### Example 4: Cloning and Tissue distribution of KCNQ5

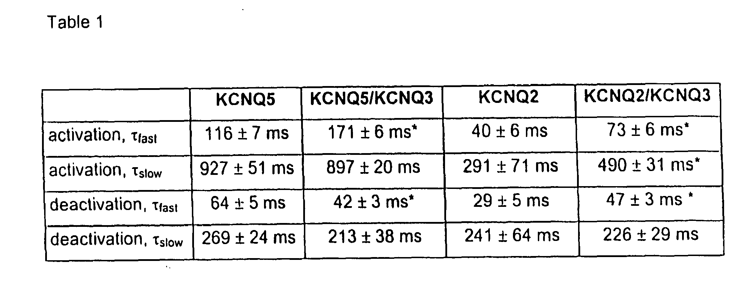

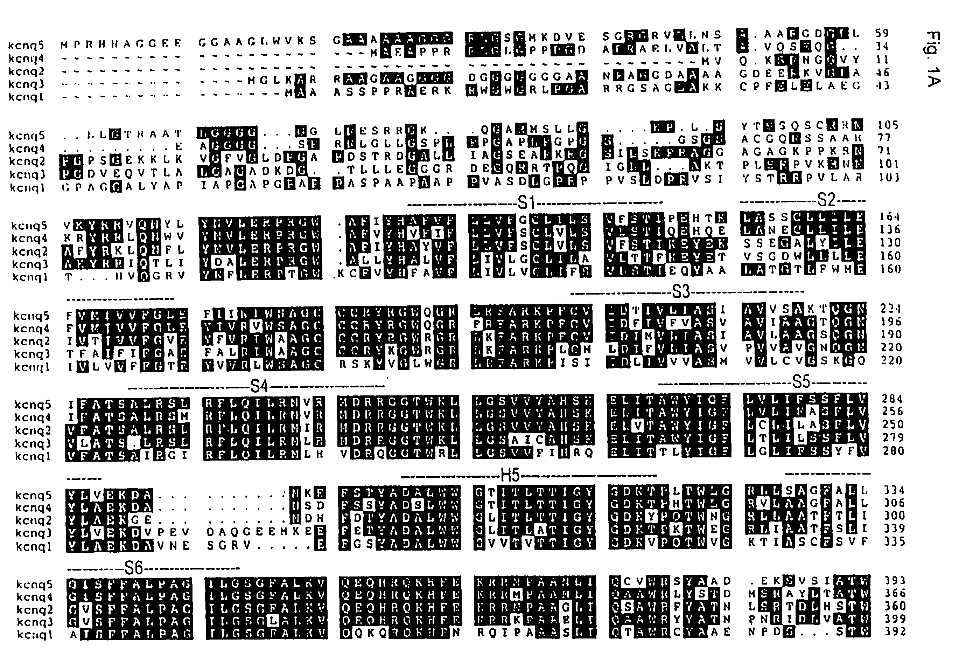

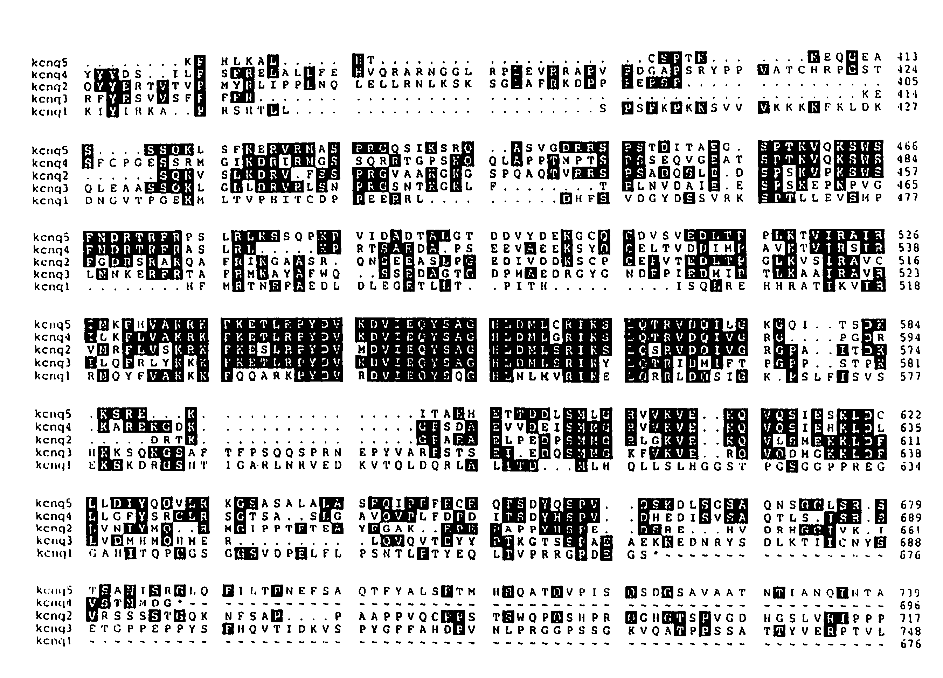

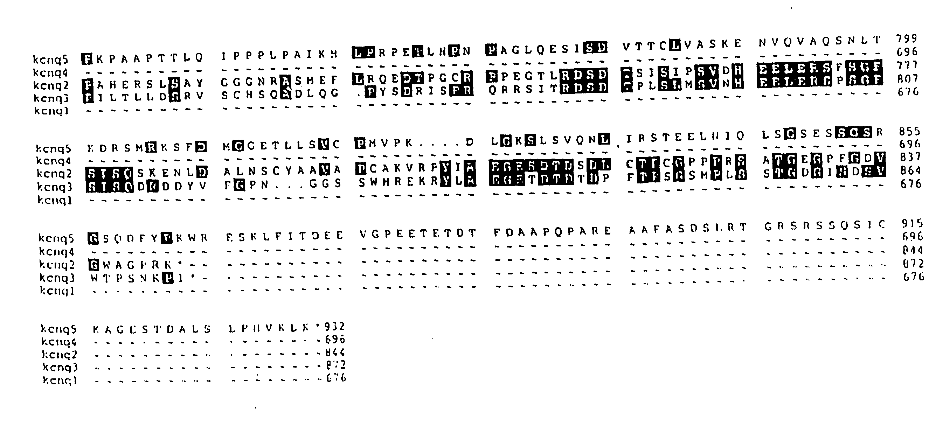

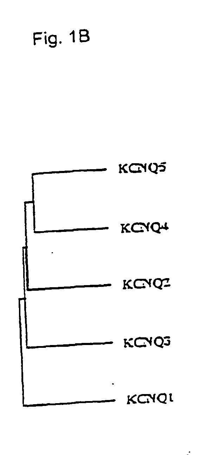

Search of the GenBank data base revealed a human BAC end sequence (AQ344243) with significant homology to the KCNQ K⁺ channel family. The sequence information was used to isolate overlapping cDNA clones from a human brain cDNA library (Edge Biosystems) and two cDNA clones were assembled to generate a full-length cDNA clone. The initiator methionine of the cDNA was assigned to the first ATG in frame, and is preceded by a stop codon in the same frame. The full-length KCNQ5 cDNA encodes a polypeptide of 932 amino acids (Fig. 1A) with a predicted molecular mass of ≈102 kDa. Hydropathy analysis supports the topology model with six transmembrane domains. KCNQ5 shows significant homology to other KCNQ proteins, with KCNQ4 being the closest relative (65% identity). KCNQ5 is about 50% identical to KCNQ3 and KCNQ2 but only 40% to KCNQ1 (Fig. 1B). Homology is observed in particular throughout the membrane spanning regions and the conserved P-loop between transmembrane segments S5 and S6. KCNQ proteins are characterized by their very long C-terminal tails. KCNQ5 has the longest C-terminus of all KCNQ proteins followed by KCNQ2 and KCNQ3. It contains a highly conserved region the function of which is still not known, but that is frequently mutated in other KCNQ proteins. Furthermore, in a BNFC family, a frame shift mutation at the nonconserved 3'end produces a mutant KCNQ2 protein with 56 additional amino acids at its C-terminus. Surprisingly, the currents generated in oocytes by the larger mutant protein were reduced by 50% compared to the wild-type. Further, introducing a stop mutation at the very same position, which truncated the last seven amino acids, increased channel activity twofold. Together these results show that the C-terminal region of KCNQ proteins is important for channel function.
The KCNQ5 protein contains numerous potential sites for phosphorylation by PKC, but lacks a N-terminal consensus site for cAMP-dependent phosphorylation that is present in KCNQ1 and KCNQ2. KCNQ1 as well as I_{Ks} currents were stimulated by PKA, whereas conflicting data have been reported concerning the effect of PKA on KCNQ2 currents.
Northern blot analyses detected 7.5 kb large KCNQ5 transcripts in human skeletal muscle and brain. In the brain transcripts are widely distributed with strongest expression in cerebral cortex, occipital pole, frontal lobe, and temporal lobe. Lower levels were detected in hippocampus and putamen. The expression pattern of KCNQ5 in the brain is very similar to that previously described for KCNQ2 and KCNQ3 that have transcripts sizes of 8.5 kb and 10.5 kb, respectively. In contrast to KCNQ2, KCNQ5 probably is absent in cerebellum.

### Example 5: Expression of KCNQ5 in Xenopus oocytes

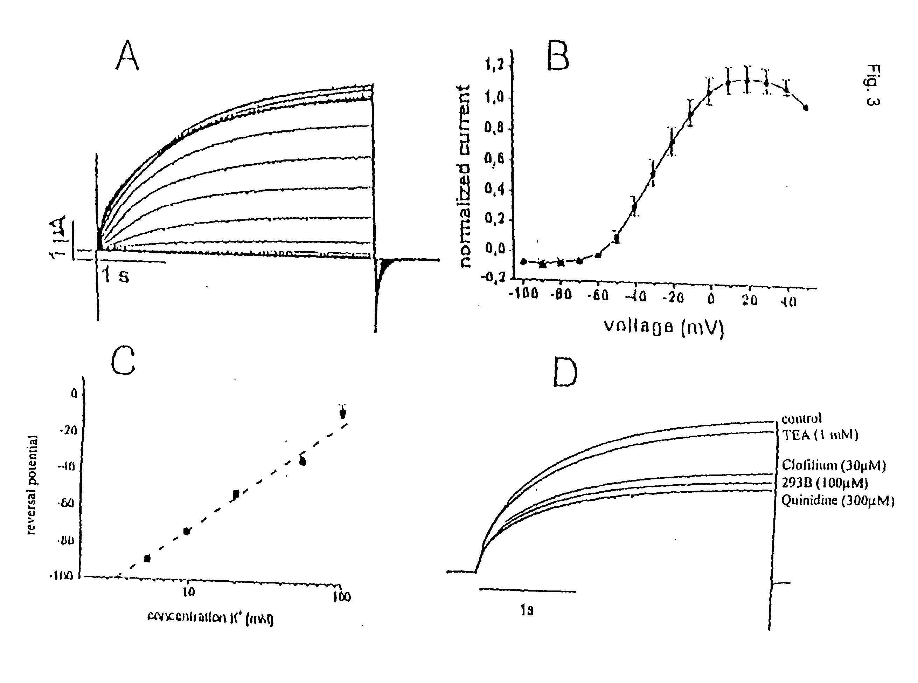

The possible function of KCNQ5 as a potassium channel was addressed by electrophysiological analysis of Xenopus oocytes injected with KCNQ5 cRNA transcribed in vitro. Two to four days after injection novel currents were detected that were not observed in water-injected control oocytes. Currents activated very slowly (Fig. 3A) and were not fully activated even within 2 seconds. At lower potentials KCNQ5 currents show a delay in activation, similar to KCNQ1 currents. Activation traces above +20 mV display a cross-over phenomenon. Activation of KCNQ5 currents is generally slower than that of other KCNQ currents. However, it can be described by a biexponential function. During a depolarizing pulse from -100 to +40 mV, current was activated with time constants of 116 ± 7, and 927 ± 51 ms (Table1). Current-voltage relations of KCNQ5 currents are shown in Fig. 3B. Currents activated at depolarizing potentials positive to -60 mV and markedly inwardly rectified at potentials greater than 0 mV. Strong inward rectification only has been demonstrated for the related KCNQ3 channels. KCNQ5 deactivation was well fitted to a second order exponential function. The two components of deactivation were determined at a repolarizing voltage of -100 mV following a 3s depolarizing step to +40 mV. Time constants were 64 ± 5 and 269 ± 24 ms (Table1). KCNQ1 tail currents display a characteristic "hook" indicative of recovery from inactivation. We did not observe such a feature at voltages between -100 and +40 mV. Additionally, a a double-pulse protocol was performed which is commonly used to reveal inactivation of potassium currents.
To examine the K⁺ selectivity of KCNQ5 currents tail current reversal potentials were determined in bath solutions containing 5.4, 9.6, 20, 54 and 96 mM K⁺. A tenfold reduction in external K⁺ shifted the reversal potential by 58 mV (Fig. 3C), indicating that KCNQ5 is perfectly selective for K⁺.
Pharmacological properties of KCNQ5 are shown in Fig. 3D. KCNQ5 currents were insensitive to the nonselective K⁺ channel blocker TEA, as reported for KCNQ3. Consistently, both channel proteins contain threonine residues within the pore region at a position that determines sensitivity to blockade by TEA. In contrast, a tyrosine residue at this position is responsible for the high sensitivity of KCNQ2 to TEA. The unspecific ion channel blocker quinidine at 300 µM blocks KCNQ5 currents by 50%.

Inhibitors of KCNQ1 and I_{Ks} were tested at concentrations that completely or strongly block these currents. From those the chromanol 293B was the most effective one, but at 100 µM it blocked KCNQ5 currents only by 45%. In comparison, at this concentration KCNQ1 is blocked by 80%. Clofilium is a class III antiarrhythmic agent and blocks KCNQ1 with an IC₅₀ <10 µM. At 30 µM it reduced KCNQ5 current by 40%, similar to its inhibitory effect on KCNQ3 (30% inhibition at 10µM). Clofilium produced little inhibition of KCNQ2 at 10µM. Thus, pharmacological properties of KCNQ5 are more similar to that of KCNQ3 than to KCNQ2.

### Example 6: Functional Interaction of KCNQ5 and KCNQ3

All members of the KCNQ family form functional heteromers with homologous or structurally different K⁺ channel subunits. To investigate the possibility that KCNQ5 might also form mixed channels with a different protein subunit, we coexpressed in Xenopus oocytes KCNQ5 with other KCNQ proteins and with cDNAs encoding different KCNE polypeptides.
After coexpression with either KCNQ1 or KCNQ2, or with one of the four known KCNE peptides (KCNE1 through KCNE4), we did not observe any evidence for functional interaction of one of those proteins with KCNQ5. However, coexpression of KCNQ5 with KCNQ3 strikingly changed resulting currents. Whereas KCNQ3 alone generated only very small currents almost indistinguishable from background level in agreement with previous findings, coinjection with KCNQ5 increased currents about 4-5 times compared with KCNQ5 alone. There was no significant shift in the voltage dependency of the currents but the I-V relationship shows that KCNQ5/KCNQ3 currents were less inwardly rectifying at positive membrane potentials. Increase in current amplitude also was accompanied by changes in gating kinetics, as shown in Table 1. Incorporation of KCNQ3 significantly slowed the fast component of activation but did not alter the slow component. Moreover, fast deactivation of KCNQS/KCNQ3 currents was slowed compared to homomeric KCNQ5. In addition to a large increase in current amplitude, association of KCNQ2 and KCNQ3 also reportedly changed gating properties of the resulting heteromeric currents. Therefore, we investigated the gating kinetics of the KCNQ2/KCNQ3 current and compared it with KCNQS/KCNQ3 (Table 1). In KCNQ2/KCNQ3 both fast and slow time constants of activation were slowed, as was the fast component of deactivation. Thus, while KCNQ2/KCNQ3 heteromers still activate faster than KCNQ3/KCNQ5 heteromers, deactivation kinetics of both heteromers were not significantly different.
The colocalization of KCNQ5 with KCNQ3 in various parts of the brain, the large increase in currents, and the differences in gating kinetics observed after coexpression strongly indicate that KCNQ5 can associate with KCNQ3 to form functional channels. Since KCNQ2/KCNQ3 channels are believed to constitute the molecular basis of the neuronal M-current, our results further suggest that KCNQ5 may also be a subunit of this physiologically important current. The M-current is expressed in peripheral and central neurons, but has not been described in the cerebellum. Consistently, KCNQ3 and KCNQ5 are only weakly or not expressed in this region, in contrast to KCNQ2 which curiously is strongly expressed in cerebellum.
KCNQ4 also can functionally interact with KCNQ3 to yield M-like currents, raising the possibility that heterogenous populations of M-currents exist within the peripheral and central nervous system that probably vary in their kinetic and pharmacological properties. Heterogeneity in the molecular composition of M-like channels may even be increased by contribution of K⁺ channels from other families.

The possibility that the KCNQ5 protein can contribute to M-currents within the brain renders the KCNQ5 gene a putative candidate for epileptic disease. By fluorescent in situ hybridization (FISH) analysis we mapped the KCNQ5 gene to chromosome 6q14. There are two loci on chromosome 6 that were linked to epileptic diseases, a known gene on 6q24, which is defect in progressive myoclonic epilepsy type 2, and an unknown gene on 6p12-p11, which is responsible for juvenile myoclonic epilepsy (EJM1). However, our mapping results exclude involvement of KCNQ5 in the latter disease. The identification of KCNQ5 makes it now possible to investigate a possible association with other forms of genetically inherited epilepsy.

For the methods used within the scope of this invention reference is made to "Current protocols of Molecular Biology; eds.: Ausubel F. M. et al.; Wiley Interscience, New York ; 2000 (currently updated)" concerning Molecular Biology and to "Experimental Biochemistry; eds.: Switzer, R. L. et al. W. H. Freeman & Co 1999" concerning Biochemistry.

In the invention , a novel member of the KCNQ family was cloned and identified.

The abbreviations used are:
cAMP, cyclic 5'-adenosine monophosphate; DIG, digoxigenin; GCG, Genetics Computer Group, Wisconsin package version 10; Iₖₛ, slow component of the cardiac delayed rectifier current; kb, kilobase; KCNQ, potassium channel of the KCNQ gene family; PHA, phytohemagglutinin; PKA, proteine kinase A; SEM, standard error of the mean; TEA, tetraethylammonium.

### Description of figures

Fig.1A: Protein sequence of KCNQ5 and comparison with other KCNQ proteins. A, alignment of human KCNQ5 with human KCNQ1, KCNQ2, KCNQ3 and KCNQ4. Identical and conserved amino acids are boxed in black and grey, respectively. The six putative transmembrane domains S1 through S6 and the pore region H5 are indicated by the stipled lines. The KCNQ5 sequence has been deposited in the EMBUGenBank database.
Fig. 1B: dendrogramm of the human KCNQ proteins, generated with the Pileup programm of the GCG software package.

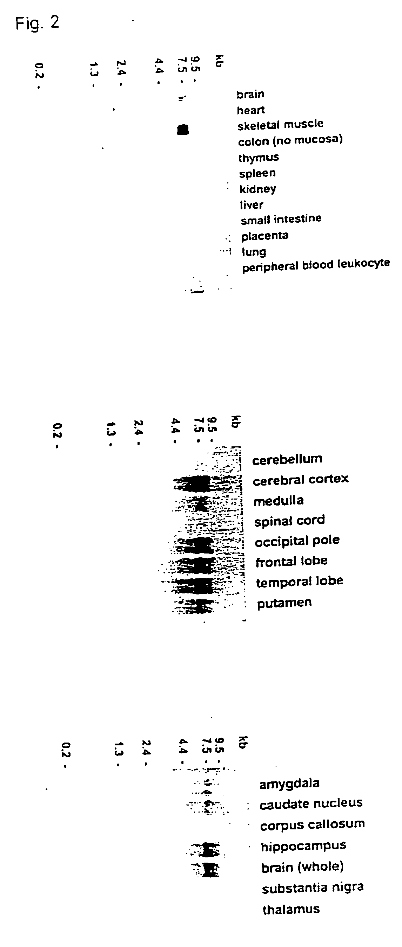

Fig. 2: Tissue distribution of human potassium channel KCNQ5. A multiple tissue Northern blot and two blots from different parts of human brain containing poly(A)⁺ RNA (Clontech) were hybridized with a KCNQ5-specific DIG-labeled RNA probe.
Fig. 3: Functional expression of KCNQ5 in Xenopus oocytes. A, representative current traces from a KCNQ5 injected oocyte subjected to a pulse protocol that stepped the membrane potential from holding potential (-100 mV) to test pulses from -100 to + 50 mV and back to holding potential. B, I-V relationships for KCNQ5 expressing oocytes (n=12) recorded using the protocol in A. C, tail current reversal potential of KCNQ5 currents as a function of extracellular K⁺ concentration. The dashed line is drawn according to the Nernst equation for a perfectly selective K⁺ channel (n=5). D, effects of TEA, clofilium, quinidine, and chromanol 293B on KCNQ5 current. Superimposed currents were obtained during a 3s depolarizing step from -100 mV to 0 mV, during the same experiment. Between compounds oocytes were perfused with ND96 until complete reversal of effects (current traces are normalized to control and averaged from 5 oocytes). Error bars indicate SEM.

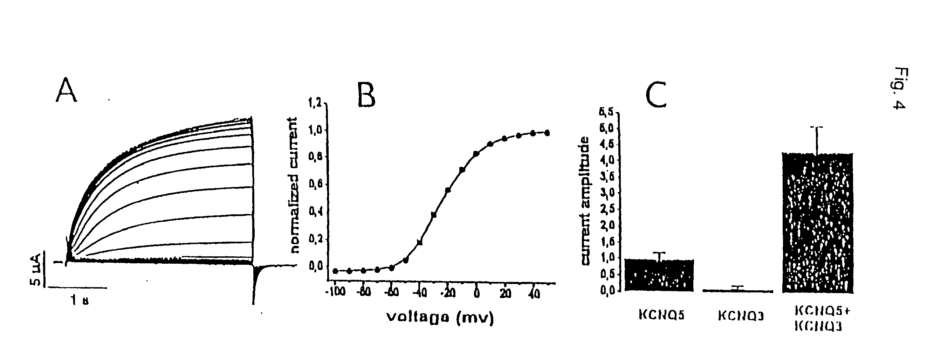

Fig. 4: Functional interaction of KCNQ5 with KCNQ3. A, representative current traces recorded from an oocyte coinjected with KCNQ5 and KCNQ3 using the same protocol as in Fig. 3A. B, I-V relationships of oocytes expressing KCNQ5 + KCNQ3 (n=11). C, bar graphs represent means of current amplitudes at the end of a 3s test pulse from -100 to +40 mV. Oocytes were injected with cRNA encoding KCNQ5 (n=14), KCNQ3 (n=6), or KCNQ5 + KCNQ3 (n=12). Error bars indicate SEM.
Fig. 5: Activation and Deactivation time constants of homomeric and heteromeric KCNQ channels. Oocytes were injected with 10 ng of either KCNQ5 or KCNQ2 cRNA, or with a mixture of 10 ng each of KCNQ5 and KCNQ3, or KCNQ2 and KCNQ3 cRNA. Currents were measured and fitted for activation and deactivation time constants as described in the text and in Experimental Procedures, respectively. Asteriks indicate significance. Values are mean ± SEM (averaged from 9-16 oocytes).

## Claims

1. A DNA sequence coding for a polypeptide exhibiting the activity of a potassium voltage gated channel and wherein the said polypeptide has the amino-acid sequence of SEQ ID No2.

2. A DNA sequence as claimed in claim 1 having the polynucleotide sequence of Seq ID No 1.

3. A recombinant DNA vector, said vector comprising
a) a polynucleotide element which renders the vector suitable for its multiplication in procaryotic or eucaryotic cells
b) a DNA sequence as claimed in one of claims 1 to 2.

4. A recombinant DNA vector as claimed in Claim 3 comprising a polynucleotide element which renders the vector suitable for multiplication in procaryotic cells in particular in cells of a bacterium.

5. A recombinant DNA vector as claimed in claim 3 or 4 wherein the cells of a bacterium are cells of Escherichia coli or Bacillus spec..

6. A recombinant DNA vector as claimed in Claim 3 comprising a polynucleotide element which renders the vector suitable for multiplication in eucaryotic cells in particular in cells of a cell line or yeast cells.

7. A recombinant DNA vector as claimed in claim 6 wherein the cells of a cell line are cells of a COS-, Hela-, or 3T3-cell-lirie and the yeast cells are cells of Saccharomyces cerevisiae..

8. A recombinant DNA vector as claimed in one of claims 3 to 7 wherein the DNA sequence as claimed in one of claims 1 to 2 is operably linked to a promotor element which allows for transcription of the related RNA and/or expression of the related protein.

9. A recombinant DNA vector as claimed in claim 8 wherein the promotor element is a procaryotic promotor.

10. A recombinant DNA vector as claimed in claim 8 wherein the promotor element is an eucaryotic promotor.

11. A recombinant DNA vector as claimed in claim 9 or 10 wherein the promotor element is inducible.

12. A host cell comprising at least one recombinant DNA vector as claimed in claims 3 to 11.

13. A host cell as claimed in claim 12 wherein the host cell is a procaryotic or eucaryotic cell.

14. A host cell as claimed in claim 13 wherein the procaryotic cell is a bacterium in particular a cell of Escherichia coli, Bacillus spec.

15. A host cell as claimed in claim 13 wherein the eucaryotic cell is a cell of a cell line in particular a cell of a COS-, a Hela-, or 3T3-cell-line or a cell of a yeast in particular a cell of Saccaromyces cerevisiae.

16. A protein encoded by one of the DNA sequences as claimed in claims 1 to 2.

17. Production of a protein as claimed in claim 16 by first growing a host cell as claimed in one of claims 12 to 15, second harvesting and processing the cells and third purifying subsequently the protein.

18. A process for identification of a compound which compound is suitable for modification of activity of a protein having activity of a potassium voltage gated channel as claimed in claim 17 comprising
a) Providing of a protein as claimed in claim 16 or 17;
b) Providing of at least one chemical compound;
c) Incubating the chemical compound according to b) with a protein according to a);
d) Measuring activity of protein according to a).

19. A process as claimed in claim 18 wherein the protein as claimed from a) is provided within a host cell as claimed in one of claims 12 to 15 and said host cell expressed the protein.

20. A process as claimed in claim 18 wherein the protein as claimed from a) is interacting with KCNQ3.

## Patentansprüche

1. DNA Sequenz, die für ein Polypeptid kodiert, welches die Aktivität eines spannungsgesteuerten Kaliumkanals aufweist, und wobei das Polypeptid die Aminosäuresequenz von SEQ ID NR. 2 aufweist.

2. DNA Sequenz nach Anspruch 1 mit der Polynucleotidsequenz von SEQ ID NR. 1.

3. Rekombinanter DNA Vektor, wobei der Vektor umfasst
a) ein Polynucleotidelement, das den Vektor für seine Multiplikation in prokaryotischen oder eukaryotischen Zellen geeignet macht,
b) eine DNA Sequenz nach einem der Ansprüche 1 bis 2.

4. Rekombinanter DNA Vektor nach Anspruch 3, umfassend ein Polynucleotidelement, das den Vektor für eine Multiplikation in prokaryotischen Zellen, insbesondere in Zellen von einem Bakterium, geeignet macht.

5. Rekombinanter DNA Vektor nach Anspruch 3 oder 4, wobei die Zellen von einem Bakterium Zellen von Escherichia coli oder Bacillus spec. sind.

6. Rekombinanter DNA Vektor nach Anspruch 3, umfassend ein Polynucleotidelement, das den Vektor für eine Multiplikation in eukaryotischen Zellen, insbesondere in Zellen einer Zelllinie oder Hefezellen, geeignet macht.

7. Rekombinanter DNA Vektor nach Anspruch 6, wobei die Zellen von einer Zelllinie Zellen von einer COS-, Hela- oder 3T3-Zelllinie sind und die Hefezellen Zellen von Saccharomyces cerevisiae sind.

8. Rekombinanter DNA Vektor nach einem der Ansprüche 3 bis 7, worin die DNA Sequenz nach einem der Ansprüche 1 bis 2 wirkungsfähig an ein Promotorelement gebunden ist, das eine Transkription der betreffenden RNA und/oder Expression des betreffenden Proteins erlaubt.

9. Rekombinanter DNA Vektor nach Anspruch 8, worin das Promotorelement ein prokaryotischer Promotor ist.

10. Rekombinanter DNA Vektor nach Anspruch 8, worin das Promotorelement ein eukaryotischer Promotor ist.

11. Rekombinanter DNA Vektor nach Anspruch 9 oder 10, worin das Promotorelement induzierbar ist.

12. Wirtszelle, umfassend mindestens einen rekombinanten DNA Vektor nach Ansprüchen 3 bis 11.

13. Wirtszelle nach Anspruch 12, worin die Wirtszelle eine prokaryotische oder eukaryotische Zelle ist.

14. Wirtszelle nach Anspruch 13, worin die prokaryotische Zelle ein Bakterium, insbesondere eine Zelle von Escherichia coli, Bacillus spec. ist.

15. Wirtszelle nach Anspruch 13, worin die eukaryotische Zelle eine Zelle von einer Zelllinie, insbesondere eine Zelle von einer COS-, einer Hela- oder 3T3-Zelllinie oder eine Zelle von einer Hefe, insbesondere eine Zelle von Saccharomyces cerevisiae, ist.

16. Protein, kodiert von einer der DNA Sequenzen nach Ansprüchen 1 bis 2.

17. Herstellung eines Proteins nach Anspruch 16 durch erstens Züchten einer Wirtszelle nach einem der Ansprüche 12 bis 15, zweitens Ernten und Verarbeiten der Zellen und drittens anschließend Reinigen des Proteins.

18. Verfahren zur Identifizierung einer Verbindung, wobei die Verbindung zur Modifizierung von Aktivität von einem Protein mit einer Aktivität eines spannungsgesteuerten Kaliumkanals nach Anspruch 17 geeignet ist, umfassend
a) Bereitstellen eines Proteins nach Anspruch 16 oder 17;
b) Bereitstellen von mindestens einer chemischen Verbindung;
c) Inkubieren der chemischen Verbindung nach b) mit einem Protein nach a);
d) Messen der Aktivität von Protein nach a).

19. Verfahren nach Anspruch 18, wobei das Protein nach a) innerhalb einer Wirtszelle nach einem der Ansprüche 12 bis 15 bereitgestellt wird und die Wirtszelle das Protein exprimiert.

20. Verfahren nach Anspruch 18, wobei das Protein nach a) mit KCNQ3 interagiert.

## Revendications

1. Séquence d'ADN codant pour un polypeptide présentant l'activité d'un canal potassique voltage dépendant et **caractérisée en ce que** ledit polypeptide a la séquence d'acides aminés de SEQ ID N° 2.

2. Séquence d'ADN selon la revendication 1, ayant la séquence polynucléotidique de SEQ ID N° 1.

3. Vecteur d'ADN recombinant, ledit vecteur comprenant
a) un élément polynucléotidique qui rend le vecteur approprié à sa multiplication dans des cellules procaryotes ou eucaryotes
b) une séquence d'ADN selon l'une des revendications 1 et 2.

4. Vecteur d'ADN recombinant selon la revendication 3, comprenant un élément polynucléotidique qui rend le vecteur approprié à la multiplication dans des cellules procaryotes, en particulier dans des cellules d'une bactérie.

5. Vecteur d'ADN recombinant selon la revendication 3 ou 4, **caractérisé en ce que** les cellules d'une bactérie sont des cellules d'Escherichia coli ou de Bacillus spec.

6. Vecteur d'ADN recombinant selon la revendication 3, comprenant un élément polynucléotidique qui rend le vecteur approprié à la multiplication dans des cellules eucaryotes, en particulier dans des cellules d'une lignée cellulaire ou des cellules de levure.

7. Vecteur d'ADN recombinant selon la revendication 6, **caractérisé en ce que** les cellules d'une lignée cellulaire sont des cellules d'une lignée cellulaire COS, Hela ou 3T3 et **en ce que** les cellules de levure sont des cellules de Saccharomyces cerevisiae.

8. Vecteur d'ADN recombinant selon l'une des revendications 3 à 7, **caractérisé en ce que** la séquence d'ADN selon l'une des revendications 1 et 2 est liée de manière opérationnelle à un élément promoteur qui permet la transcription de l'ARN associé et/ou l'expression de la protéine associée.

9. Vecteur d'ADN recombinant selon la revendication 8, **caractérisé en ce que** l'élément promoteur est un promoteur procaryote.

10. Vecteur d'ADN recombinant selon la revendication 8, **caractérisé en ce que** l'élément promoteur est un promoteur eucaryote.

11. Vecteur d'ADN recombinant selon la revendication 9 ou 10, **caractérisé en ce que** l'élément promoteur est inductible.

12. Cellule hôte comprenant au moins un vecteur d'ADN recombinant selon les revendications 3 à 11.

13. Cellule hôte selon la revendication 12, **caractérisée en ce que** la cellule hôte est une cellule procaryote ou eucaryote.

14. Cellule hôte selon la revendication 13, **caractérisée en ce que** la cellule procaryote est une bactérie, en particulier une cellule d'Escherichia coli, de Bacillus spec.

15. Cellule hôte selon la revendication 13, **caractérisée en ce que** la cellule eucaryote est une cellule d'une lignée cellulaire, en particulier une cellule d'une lignée cellulaire COS, Hela ou 3T3, ou une cellule d'une levure, en particulier une cellule de Saccharomyces cerevisiae.

16. Protéine codée par l'une des séquences d'ADN selon les revendications 1 et 2.

17. Production d'une protéine selon la revendication 16 en cultivant premièrement une cellule hôte selon l'une des revendications 12 à 15, deuxièmement en récoltant et en traitant les cellules et troisièmement en purifiant ensuite la protéine.

18. Procédé d'identification d'un composé, lequel composé convient pour modifier l'activité d'une protéine ayant une activité de canal potassique voltage dépendant selon la revendication 17, comprenant les étapes consistant à
a) fournir une protéine selon la revendication 16 ou 17;
b) fournir au moins un composé chimique;
c) incuber le composé chimique selon b) avec une protéine selon a);
d) mesurer l'activité de la protéine selon a).

19. Procédé selon la revendication 18, **caractérisé en ce que** la protéine selon a) est fournie au sein d'une cellule hôte selon l'une des revendications 12 à 15 et **en ce que** ladite cellule hôte a exprimé la protéine.

20. Procédé selon la revendication 18, **caractérisé en ce que** la protéine selon a) interagit avec KCNQ3.
